# EUROPEAN PATENT SPECIFICATION

(11) **EP 1 778 178 B1**
(45) Date of publication and mention of the grant of the patent: **15.07.2009**
(21) Application number: 05733153.0
(22) Date of filing: 29.03.2005
(51) Int. Cl.: A61K 8/64, A61Q 11/00

(54) **ORAL CARE COMPOSITIONS COMPRISING POLYASPARTATE**
MUNDPFLEGEZUSAMMENSETZUNGEN MIT POLYASPARTAT
COMPOSITIONS DE SOIN BUCCO-DENTAIRE COMPRENANT DU POLYASPARTATE

(30) Priority: 31.03.2004 GB 0407307
(43) Date of publication of application: 02.05.2007
(73) Proprietor: The Boots Company PLC, Nottingham NG2 3AA (GB); THE UNIVERSITY OF SHEFFIELD, Fifth Court Western Bank, Sheffield S10 2TE (GB)
(72) Inventor: LILLEY, Terence, Henry, Sheffield S11 7BY (GB); HAYWOOD, Julia Louise, Thorneywood, Nottingham NG3 2PZ (GB)
(74) Representative: Adamson Jones
(86) International application number: PCT/GB2005/001221
(87) International publication number: WO 2005/094765

(56) References cited:
- WO-A-99/63961
- US-A- 5 552 516
- US-A1- 2003 185 767

## Description

This invention concerns improvements relating to oral care compositions, and in particular to improvements in the performance of such compositions in respect of the inhibition, prevention and/or removal of stains upon the teeth.

Polypeptides, in particular synthetic polyamino acids, have been known for some time to be of potential utility in oral care compositions such as dentifrices, mouth washes etc.

EP-A-0305282, EP-A-0305283 and EP-A-0391629 all disclose synthetic polypeptide compounds of the general formula poly (X)ₘ(Y)ₙ, where X and Y represent different amino acid residues. The compounds are indicated to be inhibitors of mineral formation, and to be of potential utility in a number of applications including control of dental tartar. X may represent an aspartic acid (1-amino-1,2-carboxyethane) residue, and the activity of the compounds is , contrasted *inter alia* with that of polyaspartate, ie a polymer consisting entirely of aspartic acid residues, typically a 20-mer. Phosphorylation of the Y residue is indicated to increase the activity. A similar disclosure is found in WO-A-92/17194.

WO-A-92/15535 discloses that water-soluble salts of polyaspartic acid with a molecular weight range of 1000 to 5000 are effective dispersants for finely divided solid particles in aqueous suspension.

Copolymers of polyaspartic acid, and methods of their preparation, are disclosed in US-A-5408659 and US-A-5659008. The copolymers are indicated to be useful in the inhibition of dental tartar formation.

Dentifrice compositions are disclosed in WO-A-99/63961. This document is mainly concerned with improvements in the flavour of the compositions, and this is said to be achieved by the inclusion in the formulation of a non-ionic block copolymer such as a poloxamine or poloxomer. The document also mentions that the composition may contain any of numerous additives, including sources of polyphosphate or alternatives thereto that are known to be effective in reducing calcium phosphate mineral deposition related to calculus formation. Polypeptides are mentioned, in particular polyaspartic acid and polygltutamic acid.

WO-A-02/07690 describes dental composition that are said to inhibit the adherence of bacteria, plaque and stains to teeth. The compositions comprise at least one poloxamer in combination with a poly-amino acid such as polyaspartate.

Guan et al (J Appl Microbiol (2003) 94:456-461) indicates that polyaspartate reduces bacterial adhesion to hydroxyapatite surfaces, and hence that polyaspartate may be of benefit in the moderation of dental plaque development

Surprisingly, we have now found that polyaspartate is effective in inhibiting or preventing the formation of stains upon the teeth, and In the removal of existing stains, even In the absence of poloxamers and the like.

According to a first aspect of the present invention, there is provided an oral care composition comprising polyaspartate in an amount effective to inhibit or prevent the formation of, or to remove, stains upon the teeth, the composition comprising less than 0.1% w/w of non-ionic block copolymers, in particular polyoxyethylene, polyoxypropylene block copolymers and polyoxyethylene, polyoxypropylene block copolymer of ethylene diamine, wherein the polyaspartate is present at a level of between 1 and 10% w/w of the total composition.

The term "polyaspartate" means in the context of the present invention polyaspartic acid (ie a polymer of aspartic acid residues) and salts thereof. Salts of polyaspartic acid are preferably water-soluble salts, and include salts of Group I and Group II metals, particularly Group I metals, and especially the sodium salt Preferably, the polyaspartate has a molecular weight of up to 50,000, preferably 2,000 to 20,000, and more preferably 2,000 to 15,000. The polyaspartate may comprise a blend of grades of polyaspartate. A particularly preferred blend comprises equal proportions of a first form of polyaspartate having an average molecular weight in the range 2,000 to 4,000, a second form of polyaspartate having an average molecular weight in the range 4,000 to 10,000 and a third form of polyaspartate having an average molecular weight in the range 10,000 to 50,000.

Suitable grades of polyaspartate are available from Donlar Corporation, 6502 South Archer Road, Bedford Park, IL 60501. USA, and from Bayer AG, Bayerwerk, D-51368 Leverkusen, Germany.

The polyaspartate preferably comprises from about 15 to about 40 aspartic acid residues, linked by either αα- or αβ-peptide linkages.

The polyaspartate is preferably applied to the teeth in the form of an oral care composition comprising polyaspartate and a dentally acceptable carrier.

The polyaspartate is preferably present in the compositions at a level of between 0.10 and 20% wtw, more preferably 0.50 and 15% w/w, and most preferably 1 and 10% w/w of the total composition.

The compositions according to the invention are advantageous primarily in that they inhibit or prevent the formation of stains upon the teeth, or are effective in removing existing stains.

The concentration of non ionic block copolymers is less than 0.1 % w/w, or less than 0.01 % w/w, or less than 0.001% w/w.

According to another aspect of the invention there is provided a method for inhibiting or preventing the formation of stains upon the teeth, or to remove stains from the teeth, which method comprises the administration to the teeth of a human or animal subject, which teeth are stained or susceptible to staining, of an oral care composition comprising polyaspartate in an amount effective to inhibit stain formation or to remove staining, the composition comprising less than 0.1% w/w of non-ionic block copolymers, in particular polyoxyethylene, polyoxypropylene block copolymers and polyoxyethylene, polyoxypropylene block copolymers of ethylene diamine, wherein the polyaspartate is present at a level of between 1 and 10% w/w of the total composition.

As used herein, an "oral care composition" is any composition suitable and intended for administration to the buccal cavity for the purpose of improving the appearance of the teeth. The composition may, for instance, be formulated as a toothpaste, mouthrinse, toothgel, tooth paint or a dental gel.

The compositions according to the invention may, as appropriate, contain further components which are well known to those skilled in the art of oral care compositions, such as, for example, humectants, surfactants, abrasives, fluoride sources, desensitising agents, flavourings, colourings, sweeteners, antimicrobial agents to act as preservatives, bactericides and/or anti-plaque agents, structuring agents, chelating agents, whitening agents or other additional stain control agents, vitamins and any other therapeutic actives. The choice and amount of additives for any particular form of composition will be evident to, or readily determined by, those skilled in the art.

Suitable humectants for use in dentifrice compositions include polyhydric alcohols such as xylitol, sorbitol, glycerol, propylene glycol and polyethylene glycols. Sorbitol, and mixtures of glycerol and sorbitol or sorbitol and xylitol, are particularly effective. A humectant helps to prevent dentifrice compositions from hardening on exposure to air, and may also provide a moist feel, smooth texture, flowability, and a desirable sweetness in the mouth. Suitably, such humectants may comprise from about 0-85%, preferably from about 20-80% by weight of the composition.

Suitable surfactants for use in dentifrices, mouthwashes etc. are usually water-soluble organic compounds, and may be anionic, non-ionic, cationic or amphoteric species.

Anionic surfactants include the water-soluble salts of C10-C18 alkyl sulphates (eg sodium lauryl sulfates), water soluble salts of C10-C18 ethoxylated alkyl sulphates, water soluble salts of C10-C18 alkyl sarcosinates, the water-soluble salts of sulfonated monoglycerides of C10-C18 fatty acids (eg sodium coconut monoglyceride sulfonates), alkyl aryl sulfonates (eg sodium dodecyl benzene sulfonate), sodium salts of the coconut fatty acid amide of N-methyltaurine and sodium salts of long chain olefin sulfonates (eg sodium C14-C16 olefin sulfonates).

Non-ionic surfactants suitable for use in oral compositions include the products of the condensation of alkylene oxide groups with aliphatic or alkylaromatic species, and may be for example, polyethylene oxide condensates of alkyl phenols, ethylene oxide condensates of aliphatic alcohols, long chain tertiary amine oxides, long chain tertiary phosphine oxides, long chain dialkyl sulfoxides and mixtures thereof. Alternatives include ethoxylated sorbitan esters such as those available from ICI under the trade name "Tween".

Cationic surfactants are generally quaternary ammonium compounds having one C8-C18 alkyl chain and include, for example, lauryl trimethylammonium chloride, cetyl trimethylammonium bromide, cetyl pyridinium chloride, diisobutylphenoxyethoxyethyldimethylbenzylammonium chloride, coconut alkyl trimethylammonium nitrite and cetyl pyridinium fluoride.

Also useful are benzyl ammonium chloride, benzyl dimethyl stearylammonium chloride, and tertiary amines having one C1-C18 hydrocarbon group and two (poly)oxyethylene groups.

Amphoteric surfactants may be aliphatic secondary and tertiary amines comprising aliphatic species which may be branched or unbranched, and in which one of the aliphatic species is a C8-C18 species and the other contains an anionic hydrophilic group, for example, sulfonate, carboxylate, sulfate, phosphonate or phosphate. Examples of quaternary ammonium compounds are the quatemized imidazole derivatives available under the trade name 'Miranol' from the Miranol Chemical Company. Other amphoteric surfactants that may be employed are fatty acid amido alkyl betaines where one alkyl group is commonly C10-C12 such as cocamido propyl betaine, for example Tego Betain supplied by T H Goldschmidt.

Flavouring agents may be added to increase palatability and may include, for example, menthol, oils of peppermint, spearmint, wintergreen, sassafras and clove. Sweetening agents may also be used, and these include D-tryptophan, saccharin, dextrose, aspartame, levulose, acesulfam, dihydrochalcones and sodium cyclamate.

Colouring agents and pigments may be added to improve the visual appeal of the composition. Suitable colourants include dyes and pigments. A suitable and commonly used pigment is pigment grade titanium dioxide, which provides a strong white colour.

Suitably, as described above, the compositions of the invention may include a further antimicrobial agent as a preservative, antibacterial and/or anti-plaque agent. Suitable antimicrobial agents include water soluble sources of certain metal ions such as zinc, copper and silver such as zinc citrate and silver chloride, cetyl pyridinium chloride, the bis-biguanides (such as chlorhexidine), aliphatic amines, phenolics such as bromochlorophene and triclosan, salicylanilides and quaternary ammonium compounds. Optionally, the formulations may also contain enzymes that will disrupt the pellicle or interfere with bacterial intercellular polysaccharides. Examples include proteases such as papain and bromelain or dextranases. Natural enzymatic biocidal systems such as a system comprising lactoperoxidase and glucose oxidase may also be employed.

Whitening agents such as hydrogen peroxide, or materials capable of generating hydrogen peroxide such as urea (carbamide peroxide), may also be used. Additional stain control agents may also be added. Examples include sodium tripolyphosphate and pyrophosphates such a s sodium or potassium pyrophosphate.

Structuring (gelling) agents may be required in, for example, dentifrices and gums to provide desirable textural properties and "mouthfeel". Suitable agents include natural gum binders such as gum tragacanth, xanthan gum, gum karaya and gum arabic, seaweed derivatives such as Irish moss and alginates, smectite clays such as bentonite or hectorite, carboxyvinyl polymers and water-soluble cellulose derivatives such as hydroxyethyl cellulose and sodium carboxymethyl cellulose. Improved texture may also be achieved, for example, by including colloidal magnesium aluminium silicate.

Suitable vitamins for inclusion in the dental preparations of the present invention include vitamins A, B5, B6, C and E.

The composition may also comprise a polymer to enhance delivery and retention of the active ingredients to the tooth surface. Suitable polymers may include PVM/MA (poly(vinylmethylether/maleic acid)) copolymers eg Gantrez S-97 from ISP, PVP (polyvinylpyrrolidone) or PVPNA (polyvinylpyrrolidone/vinyl acetate) copolymers, eg Plasdone S-630 from ISP, and similar materials from other suppliers.

Where the composition contains any of the further ingredients or excipients mentioned above, the concentrations of such ingredients may be generally similar to those conventional in dental compositions of the corresponding form.

The efficacy of the compositions according to the invention in the removal of stains, and the inhibition of stain formation, has been demonstrated by the following methods, in which the results obtained using a standard whitening toothpaste formulation are contrasted with those obtained using the same formulation but with polyaspartate added at two different concentrations.

### Methods

### Strip Preparation

In these measurements, strips (30x10 mm) cut from 3mm thick Perspex blocks are used as model teeth. The strips were rinsed with deionised water to removes any loose material.

### Staining Solution

A staining solution of tea was prepared by infusing 1g of tea leaves (Extra Strong Tea, Marks and Spencer) in 100ml boiling water for 3 minutes. The tea solution was then passed through gauze and allowed to cool to room temperature.

### Lightness Measurement

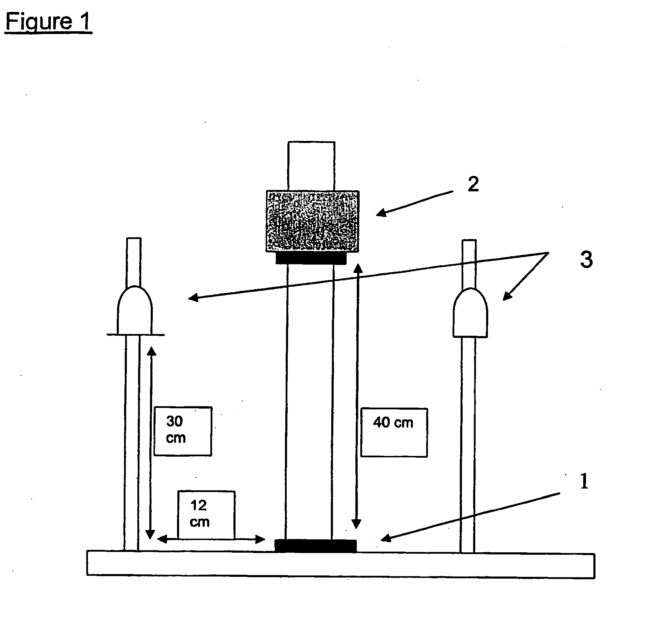

The apparatus used for lightness (L) measurement is shown schematically in Figure 1. Each Perspex strip (1) was imaged using a Kodak DCS 410 digital camera (2), mounted on the *in vitro* frame under two UV fluorescent tubes (3) and two daylight tubes. The camera was set 40cm above and perpendicular to the specimen using aperture F11 and a shutter speed of 1/10 second. The standardised lighting source comprised two UV tubes and two 6500K 'daylight' tubes which were set at a vertical distance of 30cm and a horizontal distance of 12 cm from the specimen.

Each Perspex strip was placed on a grey background, ensuring that the strip was centred in the viewfinder. A control block was imaged with every set of treated blocks. Each image was transferred to Adobe PhotoShop software and area of 200x200 pixels (8mm²) was analysed and an average lightness (L) reading obtained using the software.

### A. Stain Removal

### Pre-treatment Stain Build-Up

Six strips were used for each treatment group. A salivary pellicle was formed by immersing the strips in pooled unstimulated human saliva for 2 minutes and then briefly washing with deionised water. The strips were the n placed in a 0.2% w/v chlorhexidine gluconate solution (Corsodyl, Glaxo SmithKline) for 2 minutes and again washed with deionised water.

The strips were then placed into 10 ml of the staining solution for 1 hour at room temperature. After this staining step the strips were removed, rinsed with deionised water and then allowed to air-dry at ambient temperature. The staining cycle of saliva, chlorhexidine gluconate and staining solution was repeated until the strips had an absorbance of 1.0 or greater when measured at 395nm.

### Stain Removal Treatment

Following a baseline image analysis measurement, the pre-stained strips were immersed in 10ml of 25% w/v toothpaste slurry (15g tooth paste in 60ml deionised water) for 1 minute. The strips were washed with deionised water and allowed to air-dry. This was repeated a further 4 times to give a total of 5 minutes exposure. Image analysis measurements were taken and L values calculated. The mean L value was calculated by averaging the L values for all strips in the treatment group.

### Results

The results are given in Table 1. It can be seen that the degree of stain removal achieved using the formulations containing polyaspartate was greater than that achieved using the conventional whitening toothpaste.

**Table 1**

| Formulation | % stain removal |
|---|---|
| Conventional Whitening formula (Example 1) | 7.48 |
| Conventional whitening formula + 2% PA (Example 2) | 9.24 |
| Conventional Whitening formula + 5% PA (Example 3) | 26.38 |

### B. Stain Inhibition

Six strips were used for each treatment group. Following a baseline image analysis measurement, the strips were immersed in pooled human unstimulated saliva for 2 minutes and then briefly washed with deionised water.

The strips were exposed to a 25% w/v toothpaste slurry slurry (15g toothpaste in 60ml deionised water) for 2 minutes and again washed with deionised water. The strips were then placed in a 0.2% w/v chlorhexidine gluconate solution (Corsodyl, Glaxo SmithKline) for 2 minutes and again washed with deionised water.

The strips were placed into 10 ml of the staining solution for 1 hour at room temperature. After this staining step, the strips were removed, rinsed with deionised water and then allowed to air-dry at ambient temperature. The treatment cycle of saliva, toothpaste, chlorhexidine gluconate and staining solution was repeated for a further 2 cycles to give a total of 3 complete cycles. Image analysis measurements were taken and L values calculated. The mean L value was calculated by averaging the L values for all strips in the treatment group.

### Results

The results are given in Table 2. It can be seen that the degree of stain inhibition achieved using the formulations containing polyaspartate was greater than that achieved using the conventional whitening toothpaste.

**Table 2**

| Formulation | % Stain inhibition |
|---|---|
| Conventional whitening formula (Example 1) | 90.50 |
| Conventional Whitening formula + 2% PA (Example 2) | 92.59 |
| Conventional Whitening formula + 5% PA (Example 3) | 96.92 |

The invention will now be described in greater detail, by way of illustration only, with reference to the following non-limiting Examples:

### (Comparative) Example 1

### Conventional Whitening Formula

| Material | %w/w |
|---|---|
| Silica abrasive | 5.56 |
| Sodium tripolyphosphate | 3.00 |
| Monosodium phosphate | 1.00 |
| Disodium phosphate | 1.00 |
| Sodium fluoride | 0.32 |
| Zinc citrate | 0.50 |
| Sodium saccharin | 0.26 |
| Cellulose abrasive | 10.00 |
| Titanium dioxide | 0.75 |
| Sodium carboxy methyl cellulose | 1.00 |
| Silica thickener | 0.83 |
| Cocamidopropyl betaine | 1.30 |
| Glycerin | 40.00 |
| Bromochlorophene | 0.10 |
| Flavour | 1.00 |
| Sodium bicarbonate | 5.00 |
| Water | To 100% |

### Method of preparation

Sodium fluoride, sodium saccharin, monosodium phosphate, disodium phosphate and sodium tripolyphosphate are dissolved in water. Glycerin is added, followed by sodium bicarbonate and zinc citrate. Silica abrasive, silica thickener, cellulose abrasive, titanium dioxide, sodium carboxy methyl cellulose and cocam idopropyl betaine are added under vacuum and the bulk is mixed until homogeneous. Bromochlorophen is dissolved in flavour and added to bulk which is striired under vacuum until homogeneous.

### Example 2

### Conventional whitening toothpaste + 2% PA

| Material | %w/w |
|---|---|
| Silica abrasive | 5.56 |
| Sodium tripolyphosphate | 3.00 |
| Monosodium phosphate | 1.00 |
| Disodium phosphate | 1.00 |
| Sodium polyaspartate (40%) | 5.00 |
| [1:1:1 blend of 3 molecular weights (3,000, 5,000 and 30,000) available from Donlar Corporation, USA] | |
| Sodium fluoride | 0.32 |
| Zinc citrate | 0.50 |
| Sodium saccharin | 0.26 |
| Cellulose abrasive | 10.00 |
| Titanium dioxide | 0.75 |
| Sodium carboxy methyl cellulose | 1.00 |
| Silica thickener | 0.83 |
| Cocamidopropyl betaine | 1.30 |
| Glycerin | 40.00 |
| Bromochlorophene | 0.10 |
| Flavour | 1.00 |
| Sodium bicarbonate | 5.00 |
| Water | To 100% |

### Method of preparation

Sodium fluoride, sodium saccharin, sodium polyaspartate, monosodium phosphate, disodium phosphate and sodium tripolyphosphate are dissolved in water. Glycerin is added, followed by sodium bicarbonate and zinc citrate. Silica abrasive, silica thickener, cellulose abrasive, titanium dioxide, sodium carboxymethyl cellulose and cocamidopropyl betaine are added under vacuum and the bulk is mixed until homogeneous. Bromochlorophen is dissolved in flavour and added to bulk which is striired under vacuum until homogeneous.

### Example 3

### Conventional whitening toothpaste + 5% PA

| Material | %w/w |
|---|---|
| Silica abrasive | 5.56 |
| Sodium tripolyphosphate | 3.00 |
| Monosodium phosphate | 1.00 |
| Disodium phosphate | 1.00 |
| Sodium polyaspartate (40%) | 12.50 |
| [1:1:1 blend of 3 molecular weights (3,000, 5,000 and 30,000) available from Donlar Corporation, USA] | |
| Sodium fluoride | 0.32 |
| Zinc citrate | 0.50 |
| Sodium saccharin | 0.26 |
| Cellulose abrasive | 10.00 |
| Titanium dioxide | 0.75 |
| Sodium carboxy methyl cellulose | 1.00 |
| Silica thickener | 0.83 |
| Cocamidopropyl betaine | 1.30 |
| Glycerin | 40.00 |
| Bromochlorophene | 0.10 |
| Flavour | 1.00 |
| Sodium bicarbonate | 5.00 |
| Water | To 100% |

### Method of preparation

Sodium fluoride, sodium saccharin, sodium polyaspartate, monosodium phosphate, disodium phosphate and sodium tripolyphosphate are dissolved in water. Glycerin is added, followed by sodium bicarbonate and zinc citrate. Silica abrasive, silica thickener, cellulose abrasive, titanium dioxide, sodium carboxymethyl cellulose and cocamidopropyl betaine are added under vacuum and the bulk is mixed until homogeneous. Bromochlorophen is dissolved in flavour and added to bulk which is striired under vacuum until homogeneous.

### Example 4

### Whitening Mouthwash with 1 % PA

| Material | % w/w |
|---|---|
| Xylitol | 4.00 |
| Sodium fluoride | 0.055 |
| Cetylpyridinium chloride | 0.10 |
| Sodium saccharin | 0.04 |
| Monosodium phosphate | 0.50 |
| Disodium phosphate | 0.50 |
| Sodium polyaspartate (40%) | 2.50 |
| [1:1:1 blend of 3 molecular weights (3,000, 5,000 and 30,000) available from Donlar Corporation, USA] | |
| Polysorbate 20 | 0.20 |
| PEG 40 Hydrogenated Castor Oil | 0.18 |
| Flavour | 0.15 |
| Colour | As required |
| Water | To 100% |

### Method of preparation

Xylitol, sodium fluoride, sodium saccharin, monosodium phosphate, disodium phosphate and sodium polyaspartate are dissolved in water. Flavour is solubilised in PEG-40 hydrogenated castor oil and polysorbate 20. When homogenous, this is added to bulk and stirred until dissolved. Colour is added as required.

## Claims

1. An oral care composition comprising polyaspartate in an amount effective to inhibit or prevent the formation of, or to remove, stains upon the teeth, the composition comprising less than 0.1% w/w of non-ionic block copolymers, in particular polyoxyethylene, polyoxypropylene block copolymers and polyoxyethylene, polyoxypropylene block copolymers of ethylene diamine, wherein the polyaspartate is present at a level of between 1 and 10% w/w of the total composition.

2. The composition as claimed in Claim 1, wherein the polyaspartate is a salt of polyaspartic acid.

3. The composition as claimed in Claim 2, wherein the salt is a sodium salt.

4. The composition as claimed in any preceding claim, wherein the polyaspartate has a molecular weight of up to 50,000, preferably 2,000 to 20,000, and more preferably 2,000 to 15,000.

5. The composition as claimed in any preceding claim, wherein the polyaspartate comprises from about 15 to about 40 aspartic acid residues, linked by either αα- or αβ-peptide linkages.

6. The composition as claimed in any preceding claim, which has the form of a toothpaste, mouthrinse, toothgel, tooth paint or a dental gel.

7. The composition as claimed in Claim 6, which comprises one or more further components selected from humectants, surfactants, abrasives, fluoride sources, desensitising agents, flavourings, colourings, sweeteners, antimicrobial agents to act as preservatives, bactericides and/or anti-plaque agents, structuring agents, chelating agents, whitening agents, vitamins and any other therapeutic actives.

8. A method for inhibiting or preventing the formation of stains upon the teeth, or to remove stains from the teeth, which method comprises the administration to the teeth of a human or animal subject, which teeth are stained or susceptible to staining, of an oral care composition comprising polyaspartate in an amount effective to inhibit stain formation or to remove staining, the composition comprising less than 0.1% w/w of non-ionic block copolymers, in particular polyoxyethylene, polyoxypropylene block copolymers and polyoxyethylene, polyoxypropylene block copolymers of ethylene diamine, wherein the polyaspartate is present at a level of between 1 and 10% w/w of the total composition.

9. A method as claimed in Claim 8, wherein the composition is a composition as claimed in any one of Claims 1 to 7.

## Patentansprüche

1. Mundpflegezusammensetzung, welche Polyaspartat in einer Menge aufweist, die wirksam ist, um die Bildung von Verfärbungen auf den Zähnen zu hemmen bzw. zu verhindern oder um Verfärbungen von den Zähnen zu entfernen, wobei die Zusammensetzung weniger als 0,1% Gew./Gew. nicht-ionischer Blockcopolymere aufweist, insbesondere Polyoxyethylen, Polyoxypropylen-Blockcopolymere und Polyoxyethylen, Polyoxypropylen-Blockcopolymere von Ethylendiamin, wobei das Polyaspartat in einer Konzentration zwischen 1 und 10 % Gew./Gew. der Gesamtzusammensetzung vorhanden ist.

2. Zusammensetzung nach Anspruch 1, wobei es sich bei dem Polyaspartat um ein Salz der Polyasparaginsäure handelt.

3. Zusammensetzung nach Anspruch 2, wobei es sich bei dem Salz um ein Natriumsalz handelt.

4. Zusammensetzung nach einem der vorherigen Ansprüche, wobei das Polyaspartat ein Molekulargewicht von bis zu 50.000, vorzugsweise von 2.000 bis 20.000 und mehr bevorzugt von 2.000 bis 15.000 aufweist.

5. Zusammensetzung nach einem der vorherigen Ansprüche, wobei das Polyaspartat etwa 15 bis etwa 40 Asparaginsäurereste aufweist, die entweder durch αα- oder durch αβ-Peptidbindungen verknüpft sind.

6. Zusammensetzung nach einem der vorherigen Ansprüche, welche die Form einer Zahnpasta, eines Mundwassers, eines Zahngels, einer Zahnfarbe oder eines Dentalgels hat.

7. Zusammensetzung nach Anspruch 6, welche mindestens einen weiteren Bestandteil aufweist, der aus Befeuchtungsmitteln, Netzmitteln, Schleifmitteln, Fluoridquellen, Desensibilisierungsmitteln, Geschmacksstoffen, Farbstoffen, Süßstoffen, antimikrobiellen Substanzen, die als Konservierungsmittel, Bakterizide und/oder Antiplaque-Substanzen wirken, Strukturierungsmitteln, Chelatbildnern, Weißmachern, Vitaminen und beliebigen anderen therapeutischen Wirkstoffen ausgewählt ist.

8. Verfahren zum Hemmen oder Verhindern der Bildung von Verfärbungen auf den Zähnen oder zum Entfernen von Verfärbungen von den Zähnen, wobei das Verfahren die Auftragung einer Mundpflegezusammensetzung auf die Zähne eines Menschen oder eines Tieres umfasst, dessen Zähne verfärbt oder anfällig für Verfärbungen sind, die Polyaspartat in einer Menge aufweist, die wirksam ist, um die Bildung von Verfärbungen zu hemmen oder eine Verfärbung zu entfernen, wobei die Zusammensetzung weniger als 0,1 % Gew./Gew. nicht-ionischer Blockcopolymere aufweist, insbesondere Polyoxyethylen, Polyoxypropylen-Blockcopolymere und Polyoxyethylen, Polyoxypropylen-Blockcopolymere von Ethylendiamin, wobei das Polyaspartat in einer Konzentration zwischen 1 und 10 % Gew./Gew. der Gesamtzusammensetzung vorhanden ist.

9. Verfahren nach Anspruch 8, wobei es sich bei der Zusammensetzung um eine Zusammensetzung nach einem der Ansprüche 1 bis 7 handelt.

## Revendications

1. Une composition pour soins bucco-dentaires comprenant du polyaspartate dans une quantité efficace pour inhiber ou empêcher la formation des, ou éliminer les, taches sur les dents, la composition comprenant moins de 0,1 % poids/poids de copolymères séquencés non ioniques, notamment des copolymères séquencés d'oxyde de polyéthylène, d'oxyde de polypropylène, et des copolymères séquencés d'éthylène diamine d'oxyde de polyéthylène, d'oxyde de polypropylène, dans lesquels le polyaspartate est présent à un niveau compris entre 1 et 10% poids/poids de la composition totale.

2. La composition, selon la Revendication 1, dans laquelle le polyaspartate est un sel d'acide polyaspartique.

3. La composition, selon la Revendication 2, dans laquelle le sel est un sel de sodium.

4. La composition, selon l'une quelconque des Revendications précédentes, dans laquelle le polyaspartate a une masse moléculaire allant jusqu'à 50 000, de préférence comprise entre 2 000 et 20 000 et, de façon encore plus préférable, entre 2 000 et 15 000.

5. La composition, selon l'une quelconque des Revendications précédentes, dans laquelle le polyaspartate comprend depuis environ 15 jusqu'à environ 40 résidus d'acide aspartique, lié par des chaînes peptidiques soit αα ou αβ.

6. La composition, selon l'une quelconque des Revendications précédentes, qui a la forme d'une pâte dentifrice, d'un bain de bouche, d'un gel pour les dents, d'une peinture dentaire ou d'un gel dentaire.

7. La composition, selon la Revendication 6, qui comprend un autre composant ou plus sélectionnés parmi des humidifiants, des surfactants, des abrasifs, des sources de fluorure, des agents désensibilisateurs, des aromatisants, des colorants, des édulcorants, des agents antimicrobiens pour agir en tant qu'agents conservateurs, bactéricides et/ou anti-plaque, agents structurants, chélateurs, agents blanchissants, vitamines et tous autres actifs thérapeutiques.

8. Une méthode pour inhiber ou empêcher la formation de taches sur les dents, ou pour éliminer les taches des dents, laquelle méthode comprend l'administration aux dents d'un sujet humain ou animal, lesquelles dents sont tachées ou sensibles aux taches, d'une composition pour soins bucco-dentaires comprenant du polyaspartate dans une quantité efficace pour inhiber la formation des taches ou éliminer les taches, cette composition comprenant moins de 0, 1 % poids/poids de copolymères séquencés non ioniques, notamment des copolymères séquencés d'oxyde de polyéthylène, d'oxyde de polypropylène, et des copolymères séquencés d'éthylène diamine d'oxyde de polyéthylène, d'oxyde de polypropylène, dans lesquels le polyaspartate est présent à un niveau compris entre 1 et 10% poids/poids de la composition totale.

9. Une méthode selon la Revendication 8, dans laquelle la composition est une composition selon l'une quelconque des Revendications 1 à 7.
